# EUROPEAN PATENT SPECIFICATION

(11) **EP 2 628 458 B1**
(45) Date of publication and mention of the grant of the patent: **22.05.2019**
(21) Application number: 13154178.1
(22) Date of filing: 06.02.2013
(51) Int. Cl.: A61B 18/14, A61B 18/18, A61B 18/22

(54) **Combined thermal therapy and hydrogel with embedded stem cell treatment**
Kombinierte Wärmetherapie und Hydrogel mit eingebetteter Stammzellenbehandlung
Combinaison de thérapie thermique et hydrogel avec un traitement intégré de cellules souches

(30) Priority: 20.02.2012 US 201213400223
(43) Date of publication of application: 21.08.2013
(73) Proprietor: Covidien LP, Mansfield, MA 02048 (US)
(72) Inventor: Ross, Anthony B., Boulder, CO Colorado 80301 (US)
(74) Representative: Soames, Candida Jane

(56) References cited:
- US-A1- 2003 125 615
- US-A1- 2007 088 218
- US-A1- 2010 204 638
- US-B1- 6 193 644
- TINA VERMONDEN ET AL: "Photopolymerized Thermosensitive Hydrogels: Synthesis, Degradation, and Cytocompatibility", BIOMACROMOLECULES, vol. 9, no. 3, 21 February 2008 (2008-02-21), pages 919-926, XP055065312, ISSN: 1525-7797, DOI: 10.1021/bm7013075

## Description

### BACKGROUND

### Technical Field

The present disclosure relates generally to the field of thermal therapy medical treatments. In particular, the disclosure relates to treating a target region of tissue with thermal therapy, followed by introduction of hydrogels embedded with stem cells leading to regrowth, restructuring, and cellular reproduction of the treated region of tissue.

### Background of Related Art

Therapeutic lesions in living bodies have been accomplished for many decades by using radio-frequency (RF) and other forms of energy. The procedures have been particularly useful in the field of neurosurgery, typically where RF-ablation electrodes, usually of elongated cylindrical geometry, are inserted into a living body. A typical form of such ablation electrodes incorporates an insulated sheath from which an exposed or non-insulated tip extends.

Generally, the ablation electrode is coupled between a grounded RF power source, e.g., an electrosurgical generator (outside the body) and a reference ground or indifferent electrode, e.g., return electrode, for contacting a large surface of the body. When an RF voltage is provided between the ablation electrode and the reference ground, RF current flows from the ablation electrode through the body. Typically, the current density is very high near the tip of the ablation electrode, which heats and destroys the adjacent tissue.

Tissue ablation is a medical activity that leads to cellular death within a target region (also referred to as target tissue). Historically, this endeavor has included a series of methods, each with varying degrees of effectiveness and subsequent levels of unintended consequences including possible adverse effects to surrounding tissue. Depending on the method used for tissue ablation and any underlying pathophysiology related to the medical treatment, the patient may have remaining tissue that is damaged and in need of repair. This is due to the fact that conventionally used ablation techniques have been non-selective in that they mediate cell death with methods, such as extreme heat or cold. These methods may non- selectively and adversely affect blood vessels, nerves, and connective structures adjacent to the ablation zone. Disruption of local nerves impedes the body's natural ability to sense and regulate homeostatic and repair processes at and surrounding the ablation region.

Without the advantage of a steady introduction of new materials to a damaged area, reconstruction of the blood vessels and internal linings become retarded as redeployment of cellular materials is inefficient. Therefore, conventional ablation treatments do not leave tissue in an optimal state for self-repair in regenerating the region of tissue. However, effective development and use of stem cells is a relatively recent development and is an emerging branch of technology that offers vast potential for enhancing regenerative capacity for an organ or tissue. A stem cell may be defined as a cell capable of producing unaltered daughter cells continuously, and a cell that is also capable of producing daughter cells that have differentiated characteristics.

US 2007/088218 A1, US 6 193 644 B1 and US 2003/125615 A1 describe medical devices with combined functionality of ablating tissue and in-vivo application of therapeutic agents. Therefore, the medical devices are provided with multi-lumen tubes, wherein the tubes can deliver ablation energy and supply therapeutic agents.

### SUMMARY

The invention is defined in appended independent claims 1, preferred embodiments are described in the dependent claims.

The following presents a simplified summary of the claimed subject matter in order to provide a basic understanding of some aspects of the claimed subject matter. This summary is not an extensive overview of the claimed subject matter. It is intended to neither identify key or critical elements of the claimed subject matter nor delineate the scope of the claimed subject matter. Its sole purpose is to present some concepts of the claimed subject matter in a simplified form as a prelude to the more detailed description that is presented later.

According to one aspect of the present disclosure, an energy delivery device for administering tissue therapy is provided. The energy delivery device includes a housing and an elongated body extending distally from the housing. The energy delivery device also includes a first lumen extending a length of the elongated body, the first lumen configured to facilitate application of thermal treatment to a target region of tissue and a second lumen extending the length of the elongated body, the second lumen configured to facilitate infusion of hydrogels embedded with stem cells to the target region of tissue thermally treated. The energy delivery device further includes a third lumen extending the length of the elongated body, the third lumen configured to facilitate delivery of ultraviolet (UV) light to the target region of tissue for curing the hydrogels applied to the target region of tissue. A distal end of the elongated body is positionable in proximity to the target region of tissue. The energy delivery device is configured for successive introduction of the thermal treatment, infusion of the hydrogels, and delivery of the UV light. A system may be provided comprising a power generating source for the thermal treatment, a UV light source for the UV light and a source of hydrogel embedded with stem cells.

In another aspect of the present disclosure, the thermal treatment involves ablating at least a portion of the target region of tissue. In another exemplary embodiment, the thermal treatment involves applying at least one of radiofrequency (RF) energy, microwave energy, ultrasound energy, laser ablation, thermal ablation, electroporation, and cryotherapy to at least a portion of the target region of tissue.

In yet another aspect of the present disclosure, the target region of tissue is at least a portion of a tissue selected from the group comprising: digestive, skeletal, muscular, nervous, endocrine, circulatory, reproductive, integumentary, lymphatic, urinary, and soft tissue or a combination thereof. Additionally, the target region of tissue may be at least one of a vessel, a liver, a lung, a pancreas, and a prostate.

The stem cells are at least a portion of a cell selected from the group comprising: hematopoietic stem cells, genetically modified stem cells, smooth muscle cells, epithelial cells, endothelial cells, adult stem cells, vascular endothelial cell precursor cells, and mesodermal stromal cells or a combination thereof.

In another aspect of the present disclosure, the hydrogels include a matrix scaffolding structure configured and dimensioned for the stem cells to grow on, the hydrogels including cell adhesion and cell signals for facilitating growth of the stem cells on the matrix scaffolding structure.

In yet another aspect of the present disclosure, the elongated body includes at least one channel for circulating liquid therethrough for cooling the target region of tissue when approached by the distal end of the elongated body.

In yet another aspect of the present disclosure, configured so that additional hydrogels are introducable to the target region of tissue at subsequent time intervals following the thermal treatment.

Additionally, the hydrogels may be antimicrobial and configured to facilitate diffusion of nutrients and wastes. The hydrogels introduced to the target region of tissue may also be configured to degrade after a predetermined period of time.

In another aspect of the present disclosure, a method of administering tissue therapy is presented. The method includes the steps of providing at least one energy delivery device configured to be connected to a power source; positioning the at least one energy delivery device in proximity to a target region of tissue; applying a thermal treatment to the target region of tissue; successively introducing, within a predetermined period of time after the thermal treatment, hydrogels embedded with stem cells to the target region of tissue thermally treated; and successively delivering, within a predetermined period of time after the hydrogel introduction, ultraviolet (UV) light to the target region of tissue for curing the hydrogels.

Further scope of applicability of the present disclosure will become apparent from the detailed description given hereinafter. However, it should be understood that the detailed description and specific examples, while indicating preferred embodiments of the present disclosure, are given by way of illustration only, since various changes and modifications within the scope of the present disclosure will become apparent to those skilled in the art from this detailed description.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 illustrates a perspective view of an energy delivery device for administering tissue therapy, in accordance with the present disclosure;
FIG. 2 illustrates a side, cross-sectional view of the energy device of FIG. 1, in accordance with the present disclosure;
FIG. 3 illustrates a side view of the energy device, as well as a cross-sectional view of the elongated body, further depicting at least one channel for circulating liquid therethrough, in accordance with another embodiment of the present disclosure;
FIGS. 4A-4C illustrate cross-sectional views of deployment of thermal energy, followed by deployment of hydrogels with stem cells embedded therein, and followed by deployment of ultraviolet (UV) light onto a target region of tissue, in accordance with the present disclosure;
FIG. 5 illustrates a flowchart for administering tissue therapy by using the energy delivery device of FIGS. 1-2, in accordance with one embodiment of the present disclosure;
FIG. 6 illustrates a flowchart for administering tissue therapy by using the energy delivery device of FIG. 3, in accordance with another embodiment of the present disclosure;
FIGS. 7A and 7B illustrate matrix scaffolding structures, in accordance with embodiments of the present disclosure.

### DETAILED DESCRIPTION

Particular embodiments of the present disclosure are described hereinbelow with reference to the accompanying drawings. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Embodiments of the presently disclosed apparatus will now be described in detail with reference to the drawings, in which like reference numerals designate identical or corresponding elements in each of the several views. As used herein, the term "distal" refers to that portion of the tool, or component thereof which is further from the user while the term "proximal" refers to that portion of the tool or component thereof which is closer to the user.

As used herein, the term "microwave" generally refers to electromagnetic waves in the frequency range of 300 megahertz (MHz) (3×10⁸ cycles/second) to 300 gigahertz (GHz) (3×10¹¹ cycles/second). As used herein, the term "RF" generally refers to electromagnetic waves having a lower frequency than microwaves. The phrase "ablation procedure" generally refers to any ablation procedure, such as, for example, microwave ablation or RF ablation or microwave ablation assisted resection.

The present disclosure allows for an apparatus and method for accomplishing effective ablation followed by introduction of regenerative materials so as to increase the rate of regrowth, the rate of reconstruction, and cellular repopulation of a region following ablation. The proposed method and apparatus described herein may ultimately decrease patient recovery times in a significant number of different treatment situations through more effective regeneration. The proposed method and apparatus matches these needs and allows for an increased opportunity for regrowth in tissues through the introduction of regenerative materials that may include stem cells in combination with hydrogels. The proposed method and apparatus also provides for a treatment that may be used widely in tissues that naturally regenerate (to enhance the effectiveness and rate of regeneration), in tissues without significant natural regenerative powers, and in those with pathophysiological factors that may otherwise impede regenerations.

In another aspect of the present disclosure, the proposed method and apparatus is capable of providing a framework for tissue regeneration following the use of non-thermal tissue ablation, such as irreversible electroporation (IRE), which effectively spares structural components leaving a structure upon which regeneration may be initiated. Non-thermal IRE ablation involves ablation where the primary method of cellular disruption leading to death is mediated via electroporation (rather than factors such as effects of or responses to heating).

The present disclosure further provides for a method and apparatus that may provide ablative and regenerative therapies in a single method or apparatus, or in a simplified series of effective applications of regenerative materials so as to increase the effectiveness of treatments, provide components for cellular rebuilding and introduce factors inducing proliferative response and regrowth to advance objectives for patient recovery. The current disclosure includes a method and device for treating tissue where the device has at least one channel for release of one or more materials in a device. Regenerative materials may be released through the same probe (or same device) that is used in ablation, thus leading to ablation directly followed by introduction of regenerative materials.

The above and other purposes may be achieved using regenerative materials of various qualities, such as, those that are totipotent, pluripotent, multipotent, as well as unipotent (cells), as well as those that are autogeneic, isogeneic, allogeneic, and xenogeneic. Regenerative materials may also include a variety of cells obtained through a variety of mechanisms, having: embryonic stem cells, adult stem cells, vascular endothelial cell precursors, and mesodermal stromal cells. These cells may be obtained from the use of magnetic beads, optical sensors, electric fields, as well as dielectrophoresis. Cells within the regenerative materials also may have a variety of distinct markers, protein expressions, or genetic compositions. This variety allows for multiple purposes to be effectively met.

The method may be used when the target tissue either actually is one of the following tissues or is within the following tissues: digestive, skeletal, muscular, nervous, endocrine, circulatory, reproductive, integumentary, lymphatic, urinary, and soft tissue. The method may be used to target tissue of or within a vessel, a liver, or lung tissue. The method may also be used singularly or in combination in tissues that are in the pancreas, prostate, uterus, and brain. The method may also be used to target singularly or in combination tissues that are benign, malignant, cancerous, neoplastic, preneoplastic, or tumorous.

The targets may also include benign or malignant, cancerous, neoplastic, preneoplastic, or tumors as stand-alone targets or targets found within another tissue (such as an organ or organ system). Ablation may be performed in each of laparoscopic, percutaneous, and open surgical procedures.

Regeneration refers to at least a partial restoration of an organ or tissue or new growth by an organism of organs and tissues that have been lost, removed, or injured. Regeneration may occur through several mechanisms, including but not limited to regrowth, restructuring, and cellular repopulation. Regrowth refers to growing, developing, and gradually increasing in size, number, value, or strength. Restructuring refers to a change in cell type, organ or tissue shape, pattern, or cell type or connectivity or arrangement than was originally present. Cellular repopulation refers to development of an area starting from a group of cells that may be exogenous from another part of the body or introduced in medical or experimental procedures to cause a specific effect of growth in a damaged area. Any of the processes or regeneration may be brought about or enhanced via introduction of synthetics, exogenous materials mimicking internal, natural, agents and may be brought about by pharmacological reagents including agonists or antagonists to enhance regeneration.

Reference will now be made in detail to embodiments of the present disclosure. While certain embodiments of the present disclosure will be described, it will be understood that it is not intended to limit the embodiments of the present disclosure to those described embodiments. To the contrary, reference to embodiments of the present disclosure is intended to cover alternatives, modifications, and equivalents as may be included within the spirit and scope of the embodiments of the present disclosure as defined by the appended claims.

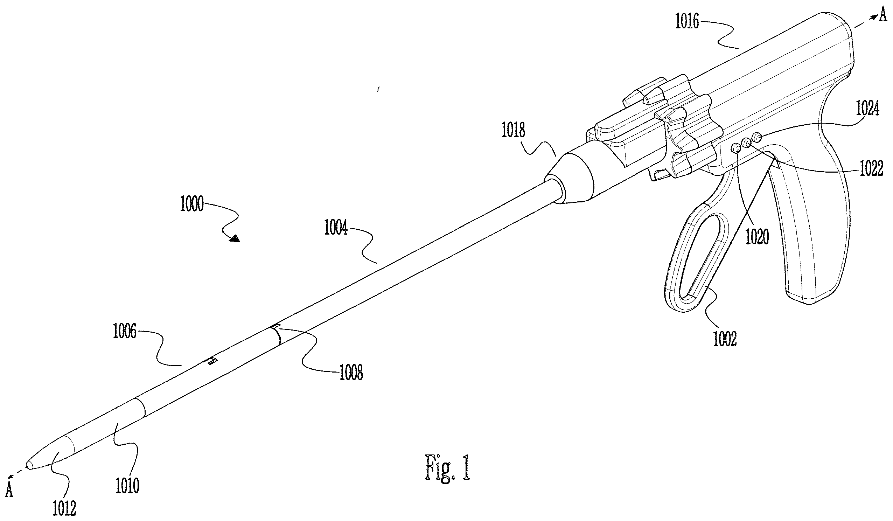

Referring to FIG. 1, a perspective view of an energy delivery device 1000 for administering tissue therapy, in accordance with the present disclosure is presented.

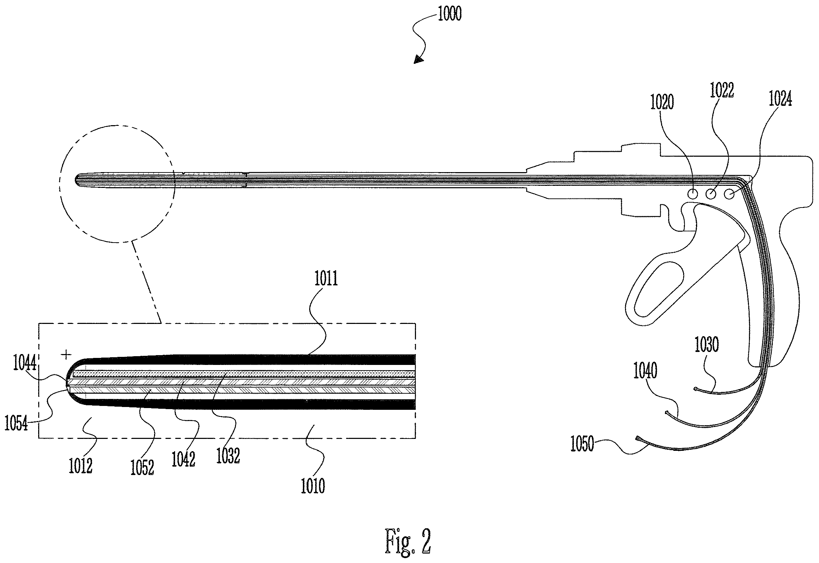

Energy delivery device 1000 is used to apply energy to patient's tissue. Energy delivery device 1000 may be configured for use, subsequent sterilization and reuse, or may be configured for single use. Energy delivery device 1000 includes a housing 1016 that has a barrel portion 1018, a movable handle 1002, an elongated shaft 1004 (defining a longitudinal axis "A-A") extending distally therefrom, and an operative tool 1006 coupled to a distal end 1008 of the elongated shaft 1004. Housing 1016 also includes a plurality of actuating mechanisms. For example, housing 1016 may include a first button 1020, a second button 1022, and a third button 1024. Actuating mechanisms 1020, 1022, 1024 will be discussed in further detail below with reference to FIG. 2.

In general, operative tool 1006 is adapted to apply energy to tissue or to a target region of tissue. Operative tool 1006 includes a distal radiating portion 1010. Distal radiating portion 1010 includes a tapered end 1012 that allows insertion into tissue with minimal resistance. It is to be understood, however, that tapered end 1012 may include other shapes, such as without limitation, a tapered end that is rounded, flat, square, hexagonal, or cylindroconical.

Referring to FIG. 2, a side, cross-sectional view of the energy delivery device 1000 of FIG. 1, in accordance with the present disclosure is presented. Three channels are depicted running the length of the housing 1016 and the elongated shaft 1004 (see FIG. 1). A first channel 1032 is connected to a first feedline 1030, a second channel 1042 is connected to a second feedline 1040, and a third channel 1052 is connected to a third feedline 1050. The first, second, and third channels 1032, 1042, 1052 may be parallel to each other and disposed within at least outer sheath 1011 of the distal radiating portion 1010 having a tapered end 1012.

The first feedline 1030 may be connected to a power generating source, such as a microwave or radio frequency (RF) electrosurgical generator (not shown). Electromagnetic energy is generally classified by increasing energy or decreasing wavelength into radio waves, microwaves, infrared, visible light, ultraviolet, X-rays and gamma-rays.

The second feedline 1040 supplies a hydrogel solution embedded with stem cells through the second channel 1042. Hydrogels are materials that absorb solvents (such as water), undergo rapid swelling without discernible dissolution, and maintain three-dimensional networks capable of reversible deformation. Hydrogels may also be utilized as a two-part adhesive system in which the hydrogel is a network of crosslinked molecules formed by reacting first and second hydrogel precursors. Hydrogels may be formed of any components within the purview of those skilled in the art.

In some embodiments, the hydrogel may be formed of a natural component, such as collagen, gelatin, serum, hyaluronic acid, combinations thereof, and the like. The natural component may degrade or otherwise be released at the site of implantation. The term "natural component" as used herein includes polymers, compositions of matter, materials, combinations thereof, and the like, which may be found in nature or derived from compositions/organisms found in nature. Natural components also may include compositions which are found in nature but may be synthesized by man, for example, using methods to create natural/synthetic/biologic recombinant materials, as well as methods capable of producing proteins with the same sequences as those found in nature, and/or methods capable of producing materials with the same structure and components as natural materials, such as synthetic hyaluronic acid, which is commercially available, for example, from Sigma Aldrich.

Stem cells have the potential to develop into many different cell types in the body during early life and growth. In addition, in many tissues, stem cells serve as a sort of internal repair system, dividing essentially without limit to replenish other cells as long as the person or animal is still alive. When a stem cell divides, each new cell has the potential either to remain a stem cell or become another type of cell with a more specialized function, such as a muscle cell, a red blood cell, or a brain cell. Stem cells are distinguished from other cell types by two important characteristics. First, stem cells are unspecialized cells capable of renewing themselves through cell division, sometimes after long periods of inactivity. Second, under certain physiologic or experimental conditions, stem cells may be induced to become tissue- or organ-specific cells with special functions. In some organs, such as the gut and bone marrow, stem cells regularly divide to repair and replace worn out or damaged tissues. In other organs, however, such as the pancreas and the heart, stem cells only divide under special conditions.

In the embodiments presented herein, the hydrogel may be coated with or include additional bioactive agents. The term "bioactive agent," as used herein, is used in its broadest sense and includes any substance or mixture of substances that have clinical use. Consequently, bioactive agents may or may not have pharmacological activity per se, e.g., a dye. Alternatively a bioactive agent could be any agent, which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. The bioactive agent may be applied to the hydrogel in any suitable form of matter, e.g., films, powders, liquids, gels and the like. Bioactive agents applied to the hydrogels may accelerate division of the stem cells embedded in the hydrogels to further facilitate healing of the target region of tissue.

The third feedline 1050 supplies UV light through the third channel 1052. The target tissue is irradiated with UV light to cure the hydrogel embedded with stem cells.

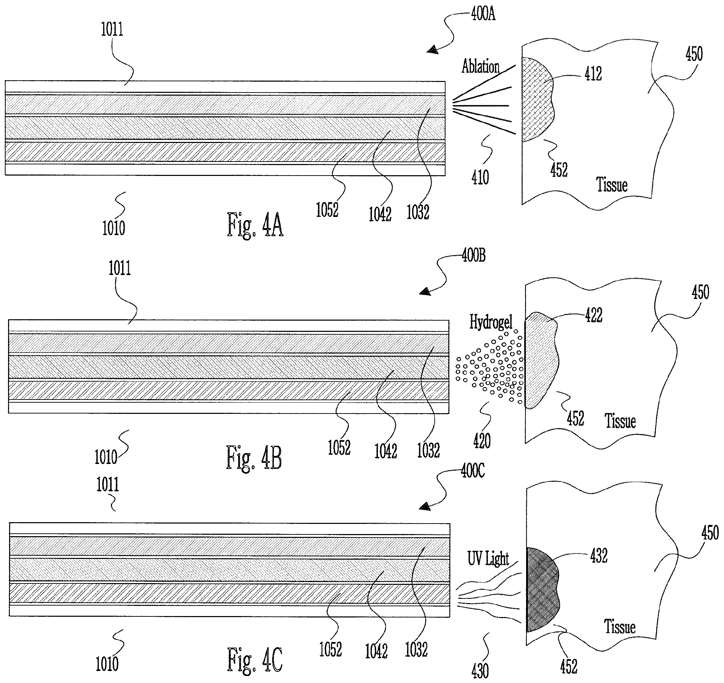

In use, the hydrogels with embedded stem cells may be emitted from a first opening 1044 located at a distal end of the distal radiating portion 1010, whereas the UV light may be emitted from a second opening 1054 located at a distal end of the distal radiating portion 1010. The first feedline 1030 may be activated via a first actuating mechanism 1020, the second feedline 1040 may be actuated via a second actuating mechanism 1022, and the third feedline 1050 may be activated via a third actuating mechanism 1024. The first, second, and third actuating mechanisms 1020, 1022, 1024 may be buttons or switches or the like positioned on, for example, the housing 1016 of energy delivery device 1000. Of course, one skilled in the art may contemplate positioning the first, second, and third actuating mechanisms 1020, 1022, 1024 on any portion or portions of energy delivery device 1000. Moreover, one skilled in the art may contemplate using a single slidable switching mechanism, instead of three buttons, in order to switch between the first, second, and third feedlines 1030, 1040, 1050. Further operation of the energy delivery device 1000 will be described below with reference to FIGS. 4A-4C.

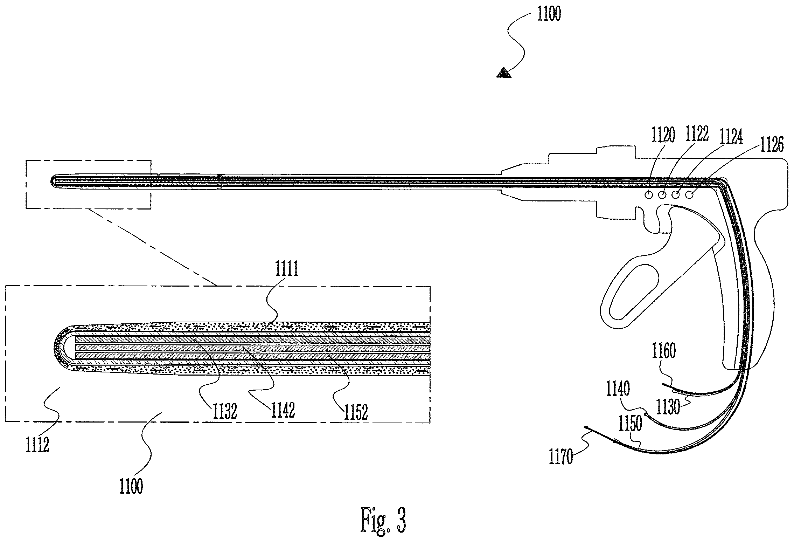

Referring to FIG. 3, a side, cross-sectional view of the energy delivery device 1100, depicting at least one channel 1113 for circulating cooling fluid 1111 therethrough, in accordance with the present disclosure is presented. FIG. 3 is of similar construction to FIG. 2, however, FIG. 3, in contrast to FIG. 2, also includes a fourth channel 1113 configured to allow a cooling fluid 1111 to flow therethrough. The fourth channel 1113 is dimensioned and adapted to extend or be disposed around the inner cylindrical surface of the distal radiating portion 1110 having a tapered end 1112. The cooling fluid 1111 may be any suitable fluid, such as without limitation, water, sterile water, deionized water, and/or saline. As in FIG. 2, a first channel 1132 is connected to a first feedline 1130, a second channel 1142 is connected to a second feedline 1140, and a third channel 1152 is connected to a third feedline 1150. Additionally, as in FIG. 2, the first feedline 1130 may be activated via a first actuating mechanism 1120, the second feedline 1140 may be actuated via a second actuating mechanism 1122, and the third feedline 1150 may be activated via a third actuating mechanism 1124.

The fourth channel 1113 having the cooling fluid 1111 flow therethrough may be in operable fluid communication with a source of cooling fluid (not shown). The source of cooling fluid is dimensioned and adapted to connect to inflow tube 1170 and outflow tube 1160. The inflow tube 1170 and the outflow tube 1160 may be formed from any suitable material, e.g., polymeric material, such as without limitation, polyimide. Inflow tube 1170 facilitates the injection of cooling fluid 1111 through fourth channel 1113 surrounding the periphery of the distal radiating portion 1110. Outflow tube 1160 facilitates the removal of cooling fluid 1111 from the fourth channel 1113 and into, for example, a collection reservoir (not shown). The inflow tube 1170 may be controlled by a button 1126 or the like positioned on the housing 1016 of the energy delivery device 1100. Of course, one skilled in the art may contemplate positioning the first, second, third, and fourth actuating mechanisms 1020, 1022, 1024, 1026 on any portion or portions of energy delivery device 1100. Moreover, one skilled in the art may contemplate using a single slidable switching mechanism, instead of four buttons, in order to switch between the first, second, and third feedlines 1030, 1040, 1050, as well as initiate the activation of the inflow tube 1170.

Referring to FIGS. 4A-4C, cross-sectional views of deployment of thermal energy, followed by deployment of hydrogels with stem cells embedded therein, and followed by deployment of ultraviolet (UV) light onto a target region of tissue, in accordance with the present disclosure are presented.

In operation, ablation energy is first delivered to a target region of tissue, as shown in FIG. 4A. After ablation has been completed, delivery of hydrogels with stem cells embedded therein takes place, as shown in FIG. 4B. Thereafter, UV light is applied to the target region of tissue to cure the stem cells embedded with hydrogels, as shown in FIG. 4C.

FIG. 4A illustrates an enhanced cross-sectional view 400A of distal radiating portion 1010 approaching tissue 450. First channel 1032 is configured to be used for the delivery of ablation energy 410. A target region 452 of tissue 450 is ablated or thermally treated with ablation energy 410. The target region 452 may receive a lethal or non-lethal dosage of ablation energy 410. In the instant case, a non-lethal dose is applied, thus forming a thermally applied target region 412.

FIG. 4B illustrates an enhanced cross-sectional view 400B of distal radiating portion 1010 approaching tissue 450. Second channel 1042 is configured to be used for the delivery of hydrogels 420 with stem cells embedded therein immediately after ablation has taken place. The thermally applied target region 412 of tissue 450 is now injected with hydrogels 420. One skilled in the art may determine the amount of hydrogels 420 to apply to the thermally applied target region 412. In the instant case, a hydrogel applied target region 422 is formed.

FIG. 4C illustrates an enhanced cross-sectional view 400C of distal radiating portion 1010 approaching tissue 450. Third channel 1052 is configured to be used for the delivery of UV light 430 to the hydrogel applied target region 422 immediately thereafter. The hydrogel applied target region 422 of tissue 450 in now cured via the delivery of UV light 430. In the instant case, the target region 452 has been transformed into a UV light applied target region 432.

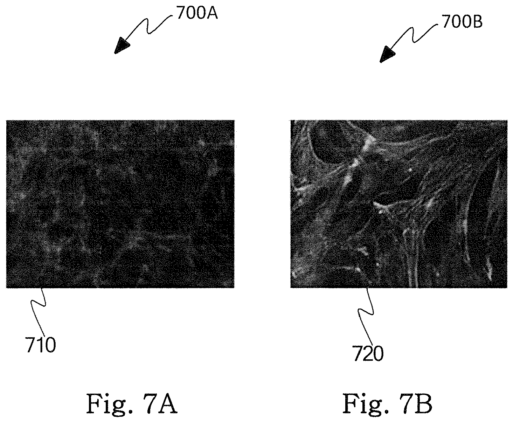

As such, successive introduction of ablation, hydrogels embedded with stem cells, and UV light is performed on a target region of tissue. There are many advantages to using hydrogels with embedded stem cells. Hydrogels may be photopolymerized, which allows the structure to be shaped temporally and spatially. The UV light may be guided to the specific location and may be shuttered on and off. Also, a hydrogel may be injected through a needle, which allows for use in minimally invasive surgeries and allows the substance to fill the space that it is injected into in three dimensions. Once injected and photopolymerized, the hydrogels may form a structure and environment for the stem cells to grow within, which mimics an extracellular matrix (see FIGS. 7A, 7B described below). Thus, insertion of the hydrogel and stem cells following ablation may allow for the tissue to re-grow effective tissue in the ablation space or area or region. The hydrogel further allows for diffusion of nutrients and wastes, and may be antimicrobial, which allows for protection against bacteria.

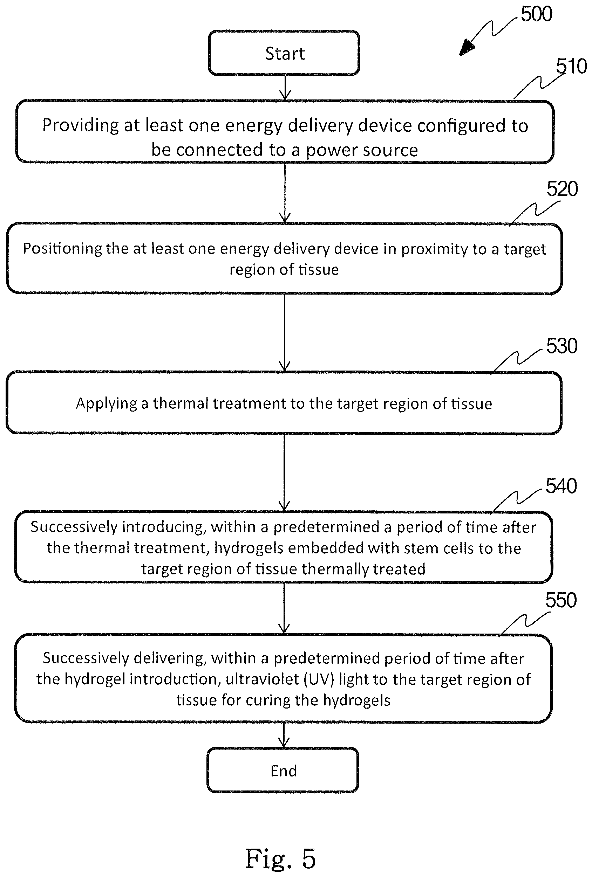

Referring to FIG. 5, a flowchart 500 for administering tissue therapy, in accordance with one embodiment of the present disclosure is presented.

The flowchart 500 includes the following steps. In step 510, at least one energy delivery device is provided to be connected to a power source. In step 520, the at least one delivery device is positioned in proximity to a target region of tissue. In step 530, thermal treatment is applied to the target region of tissue. In step 540, the user successively introduces, within a predetermined period of time after thermal treatment, hydrogels embedded with stem cells to the target region of tissue thermally treated. In step 550, the user then successively delivers, within a predetermined period of time after hydrogel introduction, UV light to the target region of tissue for curing the hydrogels. The process then ends. It is to be understood that the method steps described herein need not necessarily be performed in the order as described. Further, words such as "thereafter," "then," "next," etc. are not intended to limit the order of the steps. These words are simply used to guide the reader through the description of the method steps.

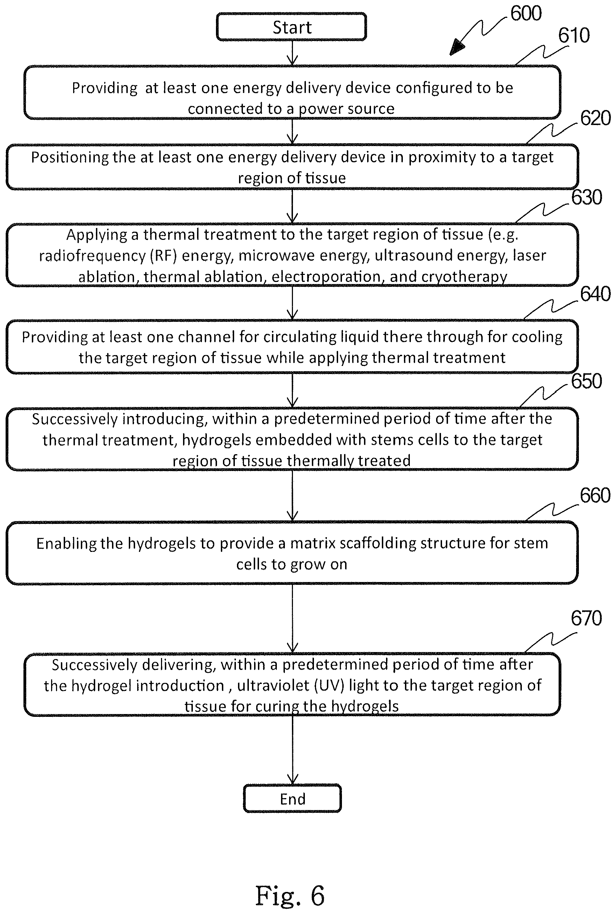

Referring to FIG. 6, a flowchart 600 for administering tissue therapy, in accordance with another embodiment of the present disclosure is presented.

The flowchart 600 includes the following steps. In step 610, at least one energy delivery device is provided to be connected to a power source. In step 620, the at least one energy delivery device is positioned in proximity to a target region of tissue. In step 630, thermal treatment is then applied to the target region of tissue (e.g., RF energy, microwave energy, ultrasound energy, laser ablation, thermal ablation, electroporation, and/or cryotherapy. In step 640, at least one channel is provided for circulating liquid therethrough for cooling the target region of tissue while applying thermal treatment. In step 650, the user thereafter successively introduces, within a predetermined period of time after the thermal treatment, hydrogels embedded with stem cells to the target region of tissue thermally treated. In step 660, the hydrogels provide a matrix scaffolding structure for stems cells to grow on. In step 670, next the user successively delivers, within a predetermined period of time after hydrogel introduction, UV light to the target region of tissue for curing the hydrogels. The process then ends. It is to be understood that the method steps described herein need not necessarily be performed in the order as described. Further, words such as "thereafter," "then," "next," etc. are not intended to limit the order of the steps. These words are simply used to guide the reader through the description of the method steps.

Referring to FIGS. 7A and 7B, matrix scaffolding structures 700A, 700B are depicted, in accordance with embodiments of the present disclosure. FIG. 7A illustrates a matrix scaffolding structure 710 after hydrogel infusion with stems cells embedded therein and FIG. 7B illustrates a matrix scaffolding structure 720 after hydrogel infusion with stem cells embedded therein. The matrix scaffolding structures 710, 720 are structures provided for the stem cells to grow on during the healing process. The hydrogel may also include cell adhesion and cell signals that encourage the growth of cells within the matrix scaffolding structures 710, 720. Moreover, the hydrogel may be engineered such that it degrades after weeks or months when the stem cells no longer need the matrix structure. One skilled in the art may contemplate a plurality of different matrix structures for permitting the growth of stem cells.

In summary, the introduction of hydrogels embedded with stem cells into a target region of tissue that has been thermally treated may improve the healing time and results immediately after thermal treatment has been applied to the target region of tissue. Also, thermal therapy may be used as an adjunct to stem cell treatments. Necrosing diseased tissue before introduction of stem cells into the region may improve the growth of stem cells because of the removal of the local cells. In certain embodiments described herein, the tissue need not be thermally necrosed, but rather a sub-lethal dose of thermal treatment may improve the treated environment for stem cell growth.

## Claims

1. A system comprising
a housing (1016);
an elongated body (1004) extending distally from the housing (1016);
a first lumen extending a length of the elongated body (1004), the first lumen configured to facilitate application of thermal treatment to a target region of tissue;
a second lumen extending the length of the elongated body (1004), the second lumen configured to facilitate infusion of hydrogels embedded with stem cells to the target region of tissue thermally treated; and
a third lumen extending the length of the elongated body (1004), the third lumen configured to facilitate delivery of ultraviolet (UV) light to the target region of tissue;
wherein a distal end of the elongated body (1004) is positionable in proximity to the target region (452) of tissue (450) for enabling successive introduction of the thermal treatment, infusion of the hydrogels, and delivery of the UV light; **characterized in** further comprising a power generating source for the thermal treatment,
a UV light source for the UV light and
a source of the hydrogel embedded with stem cells,
wherein the first lumen is connected to the power generating source for thermal treatment, the second lumen is connected to the source of the hydrogel embedded with stem cells, and the third lumen is connected to the UV light source for the UV light.

2. The system according to Claim 1, wherein the stem cells are at least a portion of a cell selected from the group comprising: hematopoietic stem cells, genetically modified stem cells, smooth muscle cells, epithelial cells, endothelial cells, adult stem cells, vascular endothelial cell precursor cells, and mesodermal stromal cells or a combination thereof.

3. The system according to Claim 1 or 2, wherein the hydrogels include a matrix scaffolding structure configured and dimensioned for the stem cells to grow on, the hydrogels including cell adhesion and cell signals for facilitating growth of the stem cells on the matrix scaffolding structure.

4. The system according to any one of claims 1 to 3, wherein the hydro gels (420) are antimicrobial and configured to facilitate diffusion of nutrients and wastes.

5. The system according to any one of claims 1 to 4, wherein the hydrogels introducable to the target region (452) of tissue (450) are configured to degrade after a predetermined period of time.

6. The system according to any one of claims 1 to 5, wherein the hydro gels are photopolymerizable hydrogels.

## Patentansprüche

1. System umfassend
ein Gehäuse (1016);
einen länglichen Körper (1004), der sich distal von dem Gehäuse (1016) erstreckt;
ein erstes Lumen, das sich über eine Länge des länglichen Körpers (1004) erstreckt, wobei das erste Lumen konfiguriert ist, um eine Anwendung einer Wärmebehandlung auf einen Zielbereich von Gewebe zu ermöglichen;
ein zweites Lumen, das sich über die Länge des länglichen Körpers (1004) erstreckt, wobei das zweite Lumen konfiguriert ist, um eine Infusion von in Stammzellen eingebetteten Hydrogelen in den Zielbereich von wärmebehandeltem Gewebe zu ermöglichen; und
ein drittes Lumen, das sich über die Länge des länglichen Körpers (1004) erstreckt, wobei das dritte Lumen konfiguriert ist, um eine Abgabe von ultraviolettem (UV-) Licht an den Zielbereich des Gewebes zu ermöglichen;
wobei ein distales Ende des länglichen Körpers (1004) in der Nähe des Zielbereichs (452) des Gewebes (450) positionierbar ist, um eine sukzessive Einführung der Wärmebehandlung, Infusion der Hydrogele und Abgabe des UV-Lichts zu ermöglichen; **gekennzeichnet durch** weiter umfassend
eine Stromerzeugungsquelle für die Wärmebehandlung,
eine UV-Lichtquelle für das UV-Licht und
eine Quelle des in Stammzellen eingebetteten Hydrogels,
wobei das erste Lumen mit der Stromerzeugungsquelle für Wärmebehandlung verbunden ist, das zweite Lumen mit der Quelle des in Stammzellen eingebetteten Hydrogels verbunden ist und das dritte Lumen mit der UV-Lichtquelle für das UV-Licht verbunden ist.

2. System nach Anspruch 1, wobei die Stammzellen zumindest ein Teil einer Zelle sind, ausgewählt aus der Gruppe, bestehend aus: hämatopoetischen Stammzellen, genetisch veränderten Stammzellen, glatten Muskelzellen, Epithelzellen, Endothelzellen, adulten Stammzellen, vaskulären Endothelzellen-Vorläuferzellen und mesodermalen Stromalzellen oder eine Kombination davon.

3. System nach Anspruch 1 oder 2, wobei die Hydrogele eine Matrixgerüststruktur aufweisen, die konfiguriert und dimensioniert ist, damit die Stammzellen darauf wachsen können, wobei die Hydrogele Zelladhäsion und Zellsignale aufweisen, um ein Wachstum der Stammzellen auf der Matrixgerüststruktur zu ermöglichen.

4. System nach einem der Ansprüche 1 bis 3, wobei die Hydrogele (420) antimikrobiell und konfiguriert sind, um eine Diffusion von Nährstoffen und Abfällen zu ermöglichen.

5. System nach einem der Ansprüche 1 bis 4, wobei die in den Zielbereich (452) des Gewebes (450) einführbaren Hydrogele konfiguriert sind, um sich nach einer vorbestimmten Zeitspanne abzubauen.

6. System nach einem der Ansprüche 1 bis 5, wobei die Hydrogele photopolymerisierbare Hydrogele sind.

## Revendications

1. Système comprenant
un boîtier (1016) ;
un corps allongé (1004) s'étendant distalement du boîtier (1016) ;
une première lumière s'étendant sur une longueur du corps allongé (1004), la première lumière étant configurée pour faciliter une application d'un traitement thermique à une région cible de tissu ;
une deuxième lumière s'étendant sur la longueur du corps allongé (1004), la deuxième lumière étant configurée pour faciliter une perfusion d'hydrogels englobés avec des cellules souches à une région cible du tissu traité thermiquement ; et
une troisième lumière s'étendant sur la longueur du corps allongé (1004), la troisième lumière étant configurée pour faciliter une distribution de lumière ultraviolette (UV) à la région cible de tissu ;
dans lequel une extrémité distale du corps allongé (1004) est positionnable à proximité de la région cible (452) de tissu (450) pour permettre une introduction successive du traitement thermique, une perfusion des hydrogels, et une distribution de la lumière UV ;
**caractérisé en outre en ce qu'**il comprend
une source de génération d'énergie pour le traitement thermique,
une source de lumière UV pour la lumière UV et
une source d'hydrogel englobé avec des cellules souches,
dans lequel la première lumière est reliée à la source de génération d'énergie pour un traitement thermique, la deuxième lumière est reliée à la source d'hydrogel englobé avec des cellules souches, et la troisième lumière est reliée à la source de lumière UV pour la lumière UV.

2. Système selon la revendication 1, dans lequel les cellules souches sont au moins une partie d'une cellule sélectionnée dans le groupe comprenant : des cellules souches hématopoïétiques, des cellules souches génétiquement modifiées, des cellules de muscle lisse, des cellules épithéliales, des cellules endothéliales, des cellules souches adultes, des cellules précurseurs de cellules endothéliales vasculaires, et des cellules stromales mésodermiques ou une combinaison de celles-ci.

3. Système selon la revendication 1 ou 2, dans lequel les hydrogels incluent une structure d'échafaudage de matrice configurée et dimensionnée pour le développement des cellules souches sur celle-ci, les hydrogels incluant des signaux d'adhérence cellulaire et des signaux cellulaires pour faciliter le développement des cellules souches sur la structure d'échafaudage de matrice.

4. Système selon l'une quelconque des revendications 1 à 3, dans lequel les hydrogels (420) sont antimicrobiens et configurés pour faciliter une diffusion de nutriments et de déchets.

5. Système selon l'une quelconque des revendications 1 à 4, dans lequel les hydrogels pouvant être introduits dans la région cible (452) de tissu (450) sont configurés pour se dégrader après une durée prédéterminée.

6. Système selon l'une quelconque des revendications 1 à 5, dans lequel les hydrogels sont des hydrogels photopolymérisables.
